# EUROPEAN PATENT SPECIFICATION

(11) **EP 3 892 063 B1**
(45) Date of publication and mention of the grant of the patent: **07.10.2026**
(21) Application number: 19828134.7
(22) Date of filing: 05.12.2019
(51) Int. Cl.: H05B 3/34, H05B 3/14

(54) **FLEXIBLE FOAM RESISTIVE HEATERS AND METHODS OF MAKING FLEXIBLE RESISTIVE HEATERS**
FLEXIBLE SCHAUMSTOFFWIDERSTANDSHEIZUNGEN UND VERFAHREN ZUR HERSTELLUNG VON FLEXIBLEN WIDERSTANDSHEIZUNGEN
CHAUFFAGES RÉSISTIFS EN MOUSSE SOUPLE ET PROCÉDÉS DE FABRICATION DE CHAUFFAGES RÉSISTIFS SOUPLES

(30) Priority: 05.12.2018 US 201862775824 P
(43) Date of publication of application: 13.10.2021
(73) Proprietor: Battelle Memorial Institute, Columbus, OH 43201-2693 (US)
(72) Inventor: PALMER, Katherine, Columbus, OH 43204 (US); HEINTZ, Amy M., Dublin, OH 43065 (US); BURTON, Brett R., Powell, OH 43065 (US); DANHOF, Scott, Plain City, OH 43064 (US); ELLIS, Jeffrey, Columbus, OH 43235 (US)
(74) Representative: Zeuner & Summerer
(86) International application number: PCT/US2019/064791
(87) International publication number: WO 2020/118117

(56) References cited:
- WO-A1-2010/143153
- DE-A1- 102007 001 132
- GB-A- 2 238 722
- US-A1- 2006 052 020
- US-A1- 2010 051 604
- US-A1- 2013 277 359
- US-A1- 2015 223 523

## Description

### Related Applications

This application claims the priority benefit of U.S. Provisional Patent Application Ser. No. 62/775,824, filed 5 December 2018.

### Introduction

Flexible resistive heaters are commonly used in consumer devices such as heating blankets. Generally, these devices incorporate heating wires, coated fabrics or printed circuits laminated between dielectric substrates. These devices can be only slightly flexible (conformal) and, for hospital use are sometimes placed under foam pads to provide suitable pressure management and minimize bed sores. The warming system should not impact the pressure management of a torso pad or headrest. While there are many thin film laminate and/or fabric heaters on the market, their rigidity impacts the pressure management system; and they also have insufficient durability to undergo repeated flexing.

Furthermore, safe warming requires a uniform and consistent temperature, generally in the range of 39 °C. Most systems place the heater under the foam pressure management system. This has several disadvantages. Thermal conduction must occur through an insulating foam. To achieve the target temperature set-point rapidly at the surface, requires high power. In addition, when pressure is applied to the foam, such as when a patient's head is placed on the foam, a hot spot occurs at the contact point. The hot spot occurs because the thickness of the insulation changes, the contact resistance between the foam and the heater changes, and/or the thermal properties of the foam change. Thus, there remains a need for improved flexible and conformal resistive heating devices for warming patients in an operating room.

There are examples in the prior art of flexible bus bar interfaces for heating elements. Augustine et al, in US 7,786,408, describes a copper braid stitched into a folded flexible heating fabric for a patient warming blanket. This patent states that the non-conductive layer comprises a non- woven polymer fabric, woven polymer fabric or cellulose material. This reference does not describe the use of a flexible compressible foam as a non-conductive layer.

US 2013/277359 A1 describes a polymer resistor produced by impregnating a meshed non-woven fabric or woven fabric with a polymer resistor. This reference does not describe a foam and does not describe a resistively-heatable CNT, graphene, or graphene oxide layer disposed on the exterior and at least partly within the foam. Document DE 10 2007 001132 A1 describes a heated car seat with at least one seat component which is provided with a seat heater, wherein the seat heater has carbon nano-tubes as an ohmic resistance.

### Summary of the Invention

In one aspect, the invention provides a method of making a resistively heatable flexible, foam, according to claim 1.

The invention also provides a resistively -heatable flexible, foam, according to claim 4. In some embodiments, a heat releasable composition is disposed in thermal contact with the resistively-heatable layer. The heat releasable composition can be adsorbed on the polymeric film or impregnated into foam such as in the cells of the foam or within channels or other voids in the foam. The invention also provides a resistively-heatable flexible, foam, comprising: a flexible, polymeric solid foam; a resistively-heatable CNT, graphene, or graphene oxide layer disposed on the exterior and at least partly within the foam; and a heat releasable composition is disposed in thermal contact with the resistively-heatable layer. The resistively heatable layer is preferably in electrical contact with electrodes. The resistively-heatable layer may comprise a dispersant or an adhesive.

In some embodiments, the resistively-heatable flexible, foam, of claim 4 further comprises at least two electrical leads attached to the foam and electrically connected to the resistively-heatable layer; and further characterizable by one or any combination of the following properties: a surface hardness, measured according to ASTM D2240 on the surface of the heatable flexible foam that is nearest the heatable layer, of 10 to 50 or 20 to 40; the leads having a contact resistance of 50 to 600 ohms, or 50 to 400 or less than 200 ohms when current is applied to the electrodes; the surface having an average surface resistance of 10 to 200 ohms, or 20 to 100 ohms; a power density of 0.1 to 0.5 W/in² about 0.2 to 0.5 W/in² ; or about 0.2 to 0.4 W/in² or about 0.2 to about 0.3 W/in² necessary to increase the average temperature of the layer from 75 °F (24 C) to 107 °F (41.7 °C); and/or where the electrical leads are separated by at least three inches or at least 5 inches and having a maximum variation of temperature between the leads and the surface of the heated area of 30 °F (16.7 °C) or less, preferably 25 °F (13.9 °C) or less, more preferably 20 °F (11.1 °C) or less, still more preferably 10 °F (5.5 °C) or less, and in some embodiments in the range of 3 to 10 °F (1.7 to 5.5 °C) when the average temperature of the layer is raised to 107 °F (41.7 °C). For example, in some preferred embodiments, the surface of the heatable flexible foam that is nearest the heatable layer, having a surface hardness of 10 to 50; or wherein the leads have a contact resistance of 50 to 400 ohms when current is applied to the electrodes sufficient to raise the temperature of the layer to 107 °F; or wherein the electrical leads are separated by at least five inches and wherein a maximum variation of temperature between the leads and a surface of the heated area is 20 °F or less when the average temperature of the layer is raised to 107 °F.

Generally, any of the inventive aspects can be defined with reference to any of the properties, including ranges of properties, that are shown in the examples. The invention includes articles having ±30% or 20% or 10% of the values shown in the examples. Likewise, any of the inventive aspects can be described in conjunction with the properties described above.

The invention also includes foams and heaters made by the inventive methods. It is believed that articles made by the inventive methods are superior to foams having CNTs uniformly dispersed in a composite material.

The invention also includes a method of warming a patient according to claim 12.

By incorporating the heating element directly onto the foam pad, we can eliminate the need for a separate thermal electric heater and the heating element is located closer to the desired location. The low viscosity of the aqueous conductive coating allows it to filter into the foam and form a 3-dimensional conductive layer on the cell walls of the foam. This 3D structure provides more points of electrical contact to the copper braid bus bar as the foam is compressed and providing an intimate contact needed for good electrical connection to occur. This electrical contact can be further improved by applying additional conductive coating (preferably the CNT, graphene or graphene oxide dispersion) over and through the lead wires.

Throughout the descriptions herein, the phrase "CNT, graphene, or graphene oxide" includes mixtures of these, or in various embodiments, any of the inventive aspects may be defined as comprising one or any combination of these. CNT (carbon nanotubes) is especially preferred. Throughout the descriptions herein, the CNT, graphene, or graphene oxide layer preferably comprises at least 50, 60, 70, 80, 90, or 95 mass% carbon. Preferably, the method includes a step of applying two electrical leads in electrical contact with the CNT, graphene, or graphene oxide layer. The electrical leads can be over (toward the external surface), under or both over and under the restively heatable layer. The electrical leads to the CNT, graphene, or graphene oxide layer can be stitched into or onto the foam wherein the stitches go through the CNT, graphene, or graphene oxide layer and into the foam, and, preferably, the stitches are nonconductive. Typically, conductive material in the CNT, graphene, or graphene oxide layer consists essentially of or is CNT and/or graphene (graphene oxide may be converted to graphene or a graphene-like conductor), and in some preferred embodiments, the conductive component of the CNT, graphene, or graphene oxide layer consists essentially of CNTs.

Preferably, the conductive coating in this invention is comprised of carbon nanotubes. Carbon nanotubes (CNTs) can be formulated into a suspension and deposited on a surface to produce a film of interpenetrating CNTs, called CNT networks. These CNT networks are of interest in applications including transparent electrodes; electrically conductive coatings for EMI shielding or lightning strike protection; and heaters for aircraft anti-icing. CNTs can also be formulated into macroscopic fibers and threads that can be used for applications including electronic textiles, non-metallic wiring, or lightning strike protection. In all these applications, the contact resistance to the bus bars is important for efficient power utilization.

The invention is often characterized by the term "comprising" which means "including," and does not exclude additional components. For example, the phrase "a dispersion comprising CNTs" does not exclude additional components. The invention includes narrower aspects in which the term "comprising" is replaced by the more restrictive terms "consisting essentially of" or "consisting of."

### Brief Description of the Drawings

Fig. 1 illustrates a foam having a surface region that is infused with a conductive coating.
Fig. 2 is a schematic illustration of foam cells coated with a conductive layer that contacts electrodes.
Fig. 3A illustrates an electric lead sewn into a conductive foam. Fig. 3B illustrates the stitches that pass through an electrode. Fig. 3C illustrates a configuration in which a foam is folded over an electrode and stitched into place.
Fig. 4 is an overhead (right) and side (left) view of a thermal fabric wrapped around electrical leads to form a heater.
Fig. 5 shows the results of hardness measurements for 4 different materials.
Fig. 6 shows the resistance at the leads (shaded bars) and surface resistance of: a prior art (Eeonyx) heatable fabric, a coated foam with leads sewn into the foams, and a coated foam with sewn threads and a coating over the stitches.
Fig. 7 shows hardness as a function of estimated contact resistance for three different resistive heaters.
Fig. 8 shows the estimated contact resistance that correlates with power to achieve a surface temperature of 107 °F (41.7 °C). For the embodiments in which the resistive layer is on the surface, the surface layer temperature and surface temperature are the same.
Fig. 9 shows hardness as a function of the power needed to achieve a surface temperature of 107 °F (41.7 °C) for four resistive heaters.
Fig. 10 shows the temperature difference between the lead and the heater for four resistive heaters.

### Detailed Description of the Invention

In a first aspect, the invention relates to devices that employ a carbon nanotube- or graphene-based conductive coating applied to flexible foam substrates. Suitable foams for these types of novel heaters are either closed, or preferably open cell foams including viscoelastic "memory" foams of any density. The coating is applied to the surface of the foam, and it absorbs into foam coating the walls of the foam cells. The coating remains flexible when dry. The optional addition of a suitable polymeric binder to the conductive coating also ensures adhesion of the conductive coating during handling and compression of the foam.

Viscoelastic foams are well-known for their comfort as mattresses, pillows (also called headrests), and seat cushions. In some preferred embodiments, the foams have one or more of the following properties: a cells/cm of 5 to 50, a density of 20 to 90 kg/m³. In some preferred embodiments, the foam is made with cell opening surfactants (for example, Ortegol 75^{®}) siloxane oils, polyether siloxanes, and/or silicone foam stabilizers. Some preferred foams are polyurethane. Residual amounts of these processing additives can be left in the foam. Flexible foams are well-known and workers skilled in this area of technology will understand that flexible foams refer to foams that flex when squeezed or compressed and generally do not crumble and recover their shape after (though not necessary immediately after) the compressive force is removed.

A resistive heating layer comprising carbon nanotubes is deposited on the flexible foam. The term "carbon nanotube" or "CNT" includes single, double and multiwall carbon nanotubes and, unless further specified, also includes bundles and other morphologies. The invention is not limited to specific types of CNTs. The CNTs can be any combination of these materials, for example, a CNT composition may include a mixture of single and multiwall CNTs, or it may consist essentially of DWNT (double-walled) and/or MWNT, or it may consist essentially of SWNT (single-walled), etc. CNTs have an aspect ratio (length to diameter) of at least 50, preferably at least 100, and typically more than 1000. In some embodiments, a CNT network layer is continuous over a substrate; in some other embodiments, it is formed of rows of CNT networks separated by rows of polymer (such as CNTs deposited in a grooved polymer substrate). The CNTs may be made by methods known in the art such as arc discharge, CVD, laser ablation, or HiPco.

In some preferred embodiments, a CNT dispersion is made by combining CNTs with glycosaminoglycans. Glycosaminoglycans are long unbranched polysaccharides consisting of a repeating disaccharide unit. The repeating unit (except for keratan) consists of an amino sugar (N-acetylglucosamine or N-acetylgalactosamine) along with a uronic sugar (glucuronic acid or iduronic acid) or galactose. Glycosaminoglycans are highly polar. Anionic glycosaminoglycans are characterized by having at some hydroxyl protons replaced by a counter ion; typically an alkali or alkaline earth element. Examples of glycosaminoglycans include: β-D-glucuronic acid, 2-O-sulfo-β-D-glucuronic acid, α-L-iduronic acid, 2-O-sulfo-α-L-iduronic acid, β-D-galactose, 6-O-sulfo-β-D-galactose, β-D-N-acetylgalactosamine, β-D-N-acetylgalactosamine-4-O-sulfate, β-D-N-acetylgalactosamine-6-O-sulfate, β-D-N-acetylgalactosamine-4-O, 6-O-sulfate, α-D-N-acetylglucosamine, α-D-N-sulfoglucosamine, and α-D-N-sulfoglucosamine-6-O-sulfate. Hyaluronan is a particularly preferred glycosaminoglycan and representative of its class.

A particularly preferred glycosaminoglycan is sodium hyaluronate. Sodium hyaluronate is the sodium salt of hyaluronic acid (HA). Materials such as sodium hyaluronate are natural products. These may be isolated from animal sources or extracted from bacteria. Hyaluron is a viscoelastic, anionic, nonsulfated glycosaminoglycan polymer (shown below). It is found naturally in connective, epithelial, and neural tissues. Its chemical structure 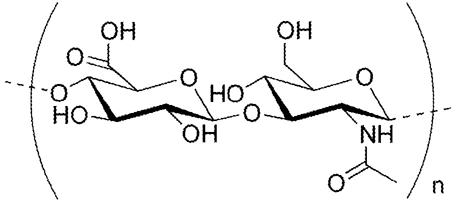 and high molecular weight make it a good dispersing agent and film former. CNT/HA aqueous dispersion and phase diagram has been reported in the literature (Moulton et al. J. Am. Chem. Soc. 2007, 129(30), 9452). These dispersions may be used to create conductive films by casting the solution onto a substrate and allowing it to dry. However, the resulting films may exhibit blistering, i.e. loss of adhesion, upon exposure to moisture or high humidity. In addition, they suffer from resistance fluctuations that occur as a result of moisture fluctuations, as HA can expand and contract, changing the junction resistance between CNT-CNT contacts. To protect the CNT/glycosaminoglycan resistive heating layer from moisture, it is desirable to cover the layer with a protective film.

A preferred CNT dispersion can be formed by combining a glycosaminoglycan such as hyaluronic acid sodium salt (HA) and carbon nanotubes (CNTs) in water. Preferably, a mass ratio in a range from about 1:1 to about 1:2 of CNTs to HA are combined in water. The loading of CNTs can be between 0.1 and 0.3 weight% to obtain a solution viscosity at 30/s in the range of 50 to 200 cP. In forming the CNT dispersion, the glycosaminoglycan may be first dissolved in a volume of water. The CNTs may then be added to the HA (or other glycosaminoglycan) solution, and additional water added to bring the suspension to a desired total volume. The suspension of CNTs in the HA solution may be sonicated to make a homogeneous dispersion of the CNTs. The CNT dispersion is applied onto a surface to form a CNT network layer over the surface. The resulting highly porous CNT layer comprises glycosaminoglycan(s) and CNTs, preferably in a mass ratio of between 3:1 to 1:3, more preferably 2:1 to 1:2, and still more preferably 1.3:1 to 1:1. Preferably, the CNT layer is then treated with an aqueous acid rinse (pH = 2.5), preferably using aqueous citric or phosphoric acid. The resulting (optionally, rinsed) film may then treated with a primer and/or a barrier layer. One preferred barrier layer is Diofan^{®} B204, which is a polyvinylidine chloride (PVDC) copolymer. Compositions such as an epoxy primer or blocked isocyantes (such as Trixene Aqua BI 220) may also serve to form the primer layer. Finally, a polymer topcoat, preferably polyurethane, is applied over the surface.

Typically, the dispersion comprises primarily water, glycosaminoglycan(s), and CNTs. In some embodiments, the dispersion comprises at least 90 mass%, or at least 95 mass% water, glycosaminoglycan(s), and carbon associated with the CNTs (i.e., CNTs and carbon impurities that may coexist with the CNTs). In some other embodiments, the dispersion may further comprise an adhesive composition (such as an acrylate) that increases adhesion of the CNTs to the foam substrate; when present, the adhesive composition preferably comprises 0.5 to 40 mass%, or 1 to 10 mass% of the dispersion. The resistive heating layer may comprise adhesive, for example, 1 to 40 or 1 to 20 or 1 to 10 mass% of the resistive heating layer may comprise one or more adhesives.

The flexible resistive heaters that are described here preferably utilize conductive CNT or graphene particles to conduct an electric current. These particles provide superior results as compared to metal foils because the particles can reversibly slide during deformation without rupturing or crinkling. Dispersions of graphene can be made as described above for CNTs or, alternatively, by other conventional methods.

For any of the descriptions in this specification, the CNT can, in alternative embodiments, be partly or fully replaced by graphene. Due to the similarity in properties between CNTs and graphene, it is believed that the substitution of graphene for CNTs would be successful. Other substitute materials could be identified through routine experimentation in view of the descriptions herein.

The use of carbon-based conductors is additionally advantageous because the resulting pads are radiolucent and a patient can be x-rayed while remaining on a pad.

The CNT coatings can be formed on the foam by solution coating processes such as spray application, screen printing, dip coating, curtain coating, gravure printing, inkjet printing, or other wet casting processes. The preferred conductive coatings are those with high bulk conductivity after drying, greater than 10 S/cm, more preferably greater than 100 S/cm. This allows the use of very thin films, less than 50 µm or more preferably less than 5 µm, as heating elements in a relatively thick foam. Surprisingly, these thin films have excellent adhesion to the foam, even during repeated stretching during compression. They do not impact the mechanical properties of the foam, so the foam still serves as a good pressure management system. Another surprising advantage of the CNT coated foams is their exceptionally high thermal uniformity (i.e., lack of hot spots) during compression and even during unequally applied compression such as when a person lies on a pad or head rest.

Another surprising result is the penetration of the highly polar CNT dispersion into the body of the hydrophobic polymeric foam. The resistance can be decreased by infusing more deeply into the foam, while still retaining only a thin layer on the cell walls. In preferred embodiments, the CNTs infuse at least 1 mm or at least 2 mm into the foam. In some preferred embodiments, the foam has a thickness of at least 1 cm, preferably at least 5 cm, and at least 90 mass% of the CNTs are in the outermost 10% or outermost 5 mm, or outermost 2 mm of the foam in the direction of thickness (not including any protective overlayer(s). The dimensions of the foam in the final article are dictated by the use of the foam; for example, where the foam is a torso pad, it will have dimensions appropriate for that use. Sheets or rolls of the material can be produced in an industrial process and cut to an appropriate size. The pre-cut sheets could have width dimensions in the range of 0.5 to 5 m, length of any size, for example at least 1 m or at least 10 m, or in the range of 0.5 to 100 m; and thickness of at least 1 cm, or least 5 cm, in some embodiments in the range of 1 to 20 cm, or 1 to 10 cm.

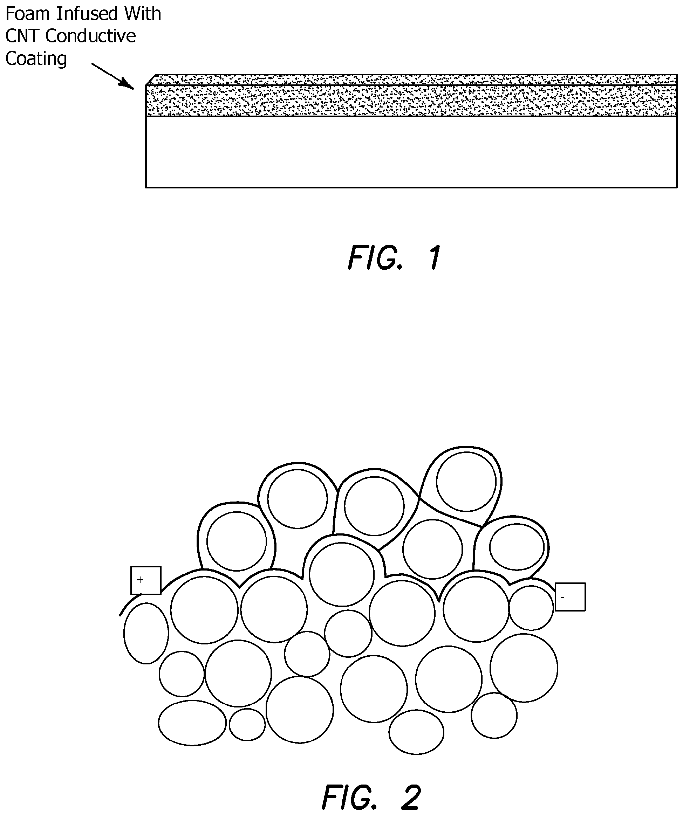

The following discussion and figure presents a theoretical description of the foam's structure. This discussion is not intended to limit the invention to these theoretical structures. The circles in Fig. 2 illustrate the walls of the open cell foam. The thicker line represents the conductive coating attached to the positive and negative leads (squares in the figure) providing the current flow. The conductive material can infuse into the foam and can coat the cell walls.

Bus bars may be applied on top of the foam or on the edges of the foam. Suitable materials for making bus bars are silver-based adhesives or inks, adhesive-backed Cu tape, or other flexible/elastomeric materials. A dielectric layer is applied as a protective coating over the top of the conductor. Suitable coatings include low durometer silicones, polyurethanes, nylons and other polymeric (polyimide, polyethylene terephthalate) as coatings or similar materials as fabrics. An example of a suitable silicone is Ecoflex 00-20, an elastomeric silicone with dielectric strength 1.4 X 107 V /mm, which preferably uses a 3.5 µm thick layer if powered at 36 V.

A resistive heater is made by connecting an electrical conductor between two electrical leads, or bus bars, and passing current through the resistive heating layer (preferably a CNT layer). The passage of current through a conductor generates heat that scales with the current (I) squared. Under most practical applications, the device is controlled by fixed voltage (V) inputs and the power generated from the heater scales as V²/R, where R is the resistance between the leads. The actual temperature measured at the surface depends on various heat transfer and heat loss mechanisms.

To provide resistive heating, a current is applied through electrical leads (also called busbars) that are connected to the CNT layer at one end and to a power source at another end. The electrical leads may be any electrically conductive material; however, conductive braids and especially copper braids are preferred. The leads may be sewn onto the surface of the flexible foam which conforms around the leads. This intimate contact ensures low contact resistance and efficient heating throughout the entire device. Surprisingly, it was discovered that a nonconductive thread can provide a low resistance electrical connection between the electrical leads and resistive heater on a flexible device, especially where the resistive heater is a flexible CNT layer disposed on the surface of a foam (especially where the resistive layer penetrates into the foam, for example, penetrates in the range of 0.2 mm to 3 mm into the foam). Nonconductive materials can be natural materials (such as cotton) or polymeric materials such as a nylon, polyurethane, polyamide, or polyester.

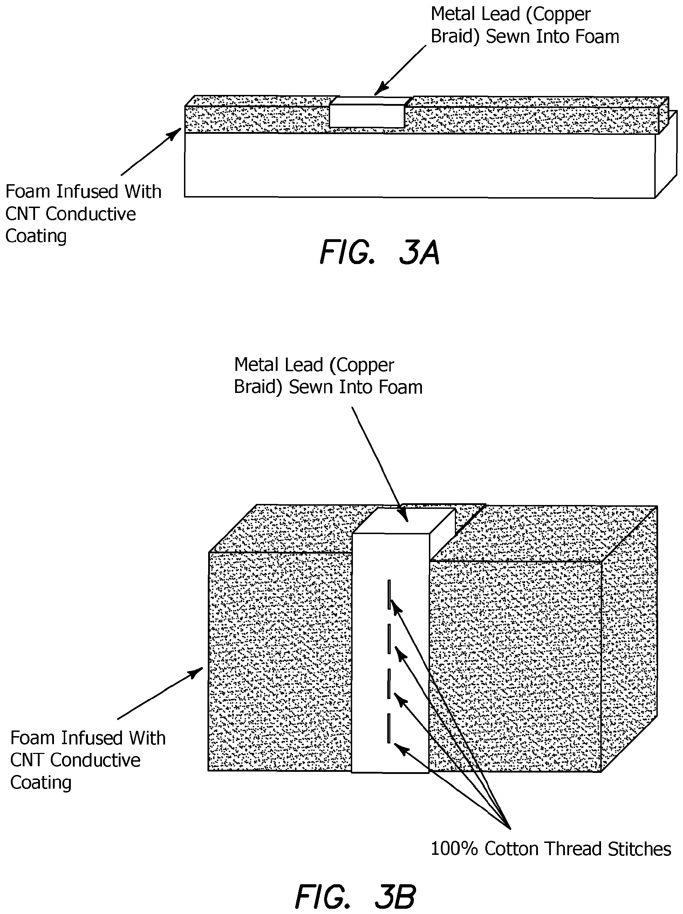

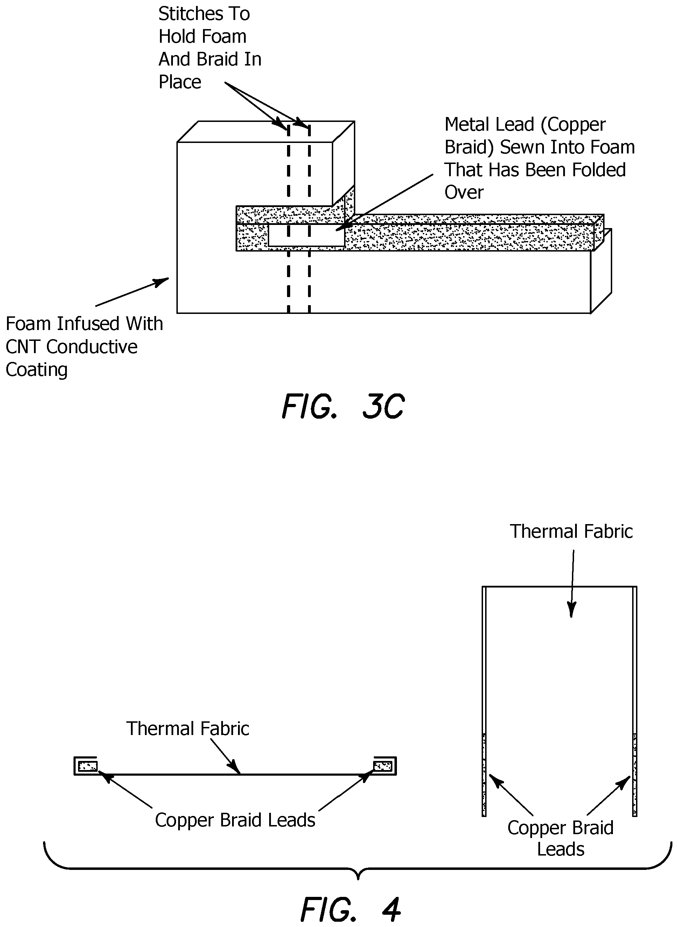

Electrical leads sown into a foam infused with a conductive coating is shown in Figs. 3A-3C. Fig. 3C illustrates a configuration in which a foam is folded over an electrode to provide intimate contact of the conductive layer with all sides around the circumference of the electrical lead.

The stitches used to hold the electrical leads to the foam are preferably made using a nonconductive thread, and can be applied using a sewing machine. Application of a conductive coating onto a foam surface can create a resistive heater. The conductive coating may be formulated with a suitable binder to ensure adequate adhesion of the coating to the foam for desired lifetime and application use. The busbars are applied either before or after the coating is applied, and located to ensure minimal effect on flexibility. An appropriate dielectric layer, such as a urethane elastomer film, is applied over conductive coating to maintain desired flexibility and to improve durability. A separate power source and controller can be selected to deliver and maintain a desired temperature.

By applying the CNT layer to a foam before the leads are applied, the coating process is simplified and the foam can be coated on a continuous roll via slot die or automatic spray process. The low viscosity CNT coating slightly infuses into the foam and coats the walls of the foam cells yet still has sufficient flexibility and adhesion when dry.

The CNT coating on the foam can be covered with an elastomeric dielectric film such as 0.001" thick polyurethane film to maintain flexibility while providing protection from electrical current. The elastomeric dielectric film is preferably at least 10 µm thick, preferably at least 20 µm thick, and in some embodiments is in the range of 10 µm to 3 mm, more preferably 10 µm to 2 mm thick.

Articles formed using the flexible resistive heater of the present invention include: headrest (pillow); blanket, mattress, OR (operating room) table, guerney cover, seat warmer (such as a car seat warmer), heatable clothing, diving undergarment, and wound healing cover.

### FABRIC SYSTEMS WITH CONDUCTIVE COATING

The advantages of the conductive coating can also be used to create fabric-based heaters, such as a scarf, which may be wrapped around the patient's head and/or neck. Fabrics can be, for example, non-woven fabrics comprised of nylon, polypropylene or other material.

### HEAT SPREADING LAYER

Heat spreaders can be used to further improve the thermal uniformity. For systems where the heater is under the foam, the addition of a heat spreading layer under or within the memory foam layer(s) (or coated fabric) can dissipate the hot spot caused when the system is nonuniformly compressed. Suitable heat spreading layers are perforated or slitted graphite or thin Al foils, thermal-filled polymers like Parker Therma-A-Gap HCS 1 O or 579, or metallized plastic. Electrically conductive heat spreaders need to be electrically isolated from the heater or there will be shorting, dielectric failure. Coated foil can be electrically insulated if a coating with the proper dielectric strength is used, such as polyethylene terephthalate, nylon or other suitable materials. Encapsulated phase change materials (PCM) such as from Microtek can be added to the dielectric coating placed on top of the CNT coatings. These materials preferably have heats of fusion that exceed 200 J/g and a melting point near 39 °C, for example in the range of 35 to 41 °C. Assuming hot spots draw 5 W/m² more heat flux than non-hot spots, a layer as thin as 0.6 mm comprising the PCM can thermally eliminate hot spots for times up to, or exceeding, 6 hours.

Delivery of Volatile Compositions - The resistive foam heaters described here can be further infused with a composition which, when heated, generates a gas or aerosol. The foam is preferably an open cell foam or a foam containing voids, or modified with channels. In some embodiments, a foam can be infused with multiple compositions; for example, compositions in separate sections (distributed by length, width, and/or thickness) of the foam. Volatilization (either with or without reaction) of the various compositions can be controlled by applying heat in selected sections; thus, the multiple compositions can be volatilized simultaneously, sequentially, or individually addressable. The CNT layer can be coated with a dielectric layer or layers that have a surface that adsorbs a desired compound. Alternatively, or in addition, the CNT-coated foam can be infused with a composition and then covered with a perforated or permeable coating. The composition to be adsorbed onto the surface or infused into the CNT-coated foam can be, for example, a fragrance, drug, prodrug, or insecticide. In the case of a fragrance, the electrical leads to the foam can be connected to electrical prongs that can be plugged into a wall socket. In another embodiment, the gas or aerosol may comprise a chemoattractant, such as octenol, that attracts a pest to the foam where it is then killed, for example by heat or electricity.

### Examples

### Example 1:

A conductive dispersion was prepared by dispersing unpurified OCSiAl single wall carbon nanotubes (SWNTs) in a solution of sodium hyaluronate using ultrasonication. The resulting dispersion had a sheet resistance of 1.5 *Ω*/square when cast as a droplet on glass. This dispersion was diluted with water and spray applied to a viscoelastic foam. Bus bars were applied by attaching copper braided leads to the foam surface with conductive silver epoxy.

Example 2. The dispersion from Example 1 was used to infuse an entire foam substrate. The foam was immersed in the dispersion, removed and air dried. The coating completely coated the cell walls, forming an interconnecting conductive pathway through the foam. Silver epoxy bus bars were added along two opposing edges on one of the square faces. Leads were soldered to conductive copper tape, which were in turn adhered to the silver bus bars. To improve conductivity between the copper tape and bus bars, a push pin was used to create several small punctures in the tape, pushing it down into the silver epoxy and foam. The resistance at the leads, uncompressed, indicate the sheet resistance was approximately 25 Ω/square. The sample generated the target heat at 5 V.

Example 3. A foam substrate was prepared be applying busbars of copper braided leads attached to the foam surface with conductive silver epoxy. CNT dispersion (OCSiAl water based Tubali Coat) was spray applied to top surface of memory foam. Two samples were prepared and tested for thermal and pressure performance. 8" X 8" samples and a 20" X 20" sample.

For thermal testing, a free-standing, precured silicone film silicone dielectric film was placed over top of the sample. Thermal imaging with an IR camera showed that the thermal uniformity is excellent. Measurement of the temperature at the surface using thermocouples separated by several inches showed that the temperature uniformity is ±0.4 °C. Thermal imaging indicated a temperature range over the entire sheet (excluding the edges) was highly uniform in the range of about 30 to 31 °C. The difference between temperature in an uncompressed and compressed state was determined by testing in an adiabatic box, where the CNT heater was used to warm the sample. The temperature difference between the steady state value with heater at 39°C and 15 minutes after putting 86.5 mmHg pressure on an area was less than 2 °C. In contrast, the temperature difference for systems where the heater was placed underneath the foam was greater than 3 °C.

Differences in pressure management were measured for a heater as prepared in Example 3, either with a free-standing silicone dielectric or a case where the heater was coated with the silicone dielectric.

### Example 4 (contemplated example):

Another embodiment of this heater may be with the addition of a binder resin to the conductive coating to achieve desired adhesion to the polyurethane foam. A water based conductive coating would be compatible with various waterbased polymer emulsions such as polyurethanes and acrylics for adhesion improvement.

Example 5. The CNT dispersion was applied to the inside of a Nylon fabric, which had a hydrophobic surface treatment. The coating was attached to copper foil bus bars. In this embodiment, the patient would be in contact with the hydrophobic outer portion. The coated fabric maintains good flexibility during handling.

### Attaching Leads - Comparative Example - Eeonyx Fabric Heater (similar to US 7,786,408)

A 6.5" X 6.5" size of Eeonyx thermal warming fabric was prepared into a resistive heater by sewing HexWik W55 copper braids onto two sides. The fabric was creased to create a ¼" fold, the copper braid was placed into the fold and a single line of cotton thread stitching held the lead in place. See Fig. 4.

### Example - Coated Foam with Sewn Leads plus coating over leads

A 20" X 40" X 0.25" piece of polyurethane viscoelastic memory foam was coated with an aqueous CNT dispersion and polyurethane binder formulation (47.6 wt % Tuball Coat E + 47.6 wt % deionized water + 4.8 wt% Helmsman Urethane) then allowed to air dry for 16 hours. This was cut into a 6.5" X 6.5" piece and copper braid (HexWik W55) was sewn onto two sides with 100% cotton thread (Gutermann CA 02776) to create a 6" X 6" heater area. An additional coat of CNT/polyurethane was applied again over the sewn copper leads for added electrical contact.

### Example - Coated Foam with Sewn Leads

A 20" X 40" X 0.25" piece of polyurethane memory foam was coated with an aqueous CNT dispersion and polyurethane binder formulation (47.6 wt % Tuball Coat E + 47.6 wt % deionized water + 4.8 wt% Helmsman Urethane) then allowed to air dry four 16 hours. This was cut into a 5.5" X 5.5" piece and copper braid (HexWik W55) was sewn onto two sides with 100% cotton thread to create a 5" X 5" heater area. No additional CNT/polyurethane was applied over this example.

### Tests

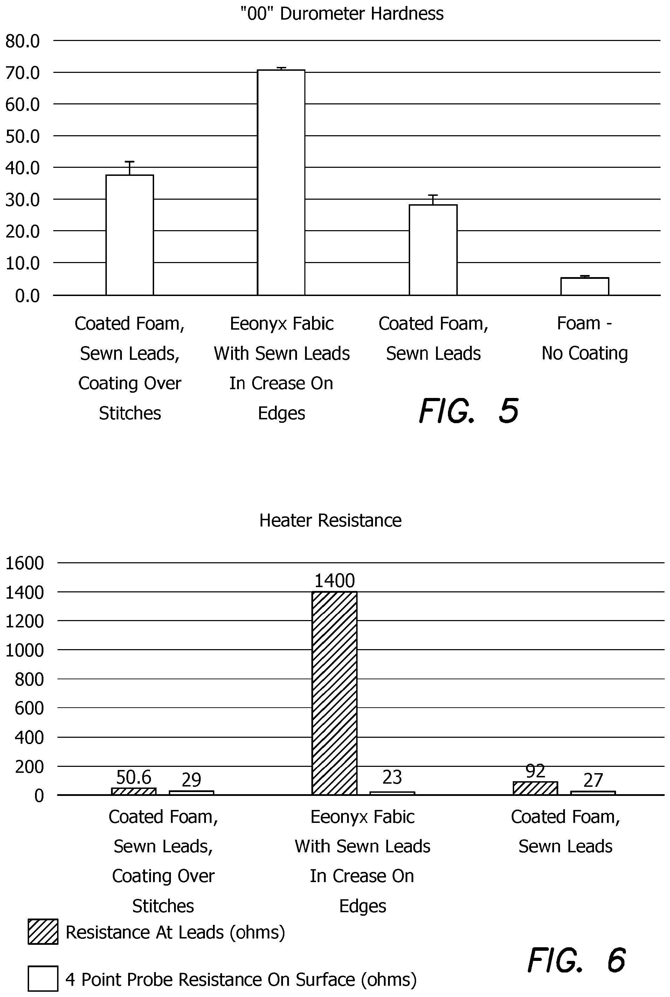

The "00" durometer hardness of each coated foam samples and an uncoated foam sample was tested per ASTM D2240 using a Rex^{®} Model DD-4 digital Durometer mounted on a OS-1 stand. Averages and standard deviation calculated from durometer readings taken in 5 unique areas of the heater. This equipment provides a maximum durometer reading to a 0.1 resolution. All tests were performed on a ¼" thick foam. The Eeonyx heater was placed on top of the foam for a comparison of the hardness. The graph below shows the results. While the conductive coating increases the hardness of the foam slightly, the Eeonyx fabric is significantly harder and less flexible than the coated and uncoated foams. See Fig. 5.

The surface resistance of the heaters was measured using a 4 point probe. This result can be compared to the resistance measured at the copper leads using a Fluke ohm meter. When the copper leads have poor electrical contact to the heater surface, the heating efficiency will be poor. Contact resistance is estimated to be the increase in resistance between the surface measurement and the lead measurement. Ideally, the surface resistance and lead resistance measurements will have little difference and the contact resistance will be very low. The samples were tested for both surface resistance and lead resistance. The results are in the graph shown in Fig. 6.

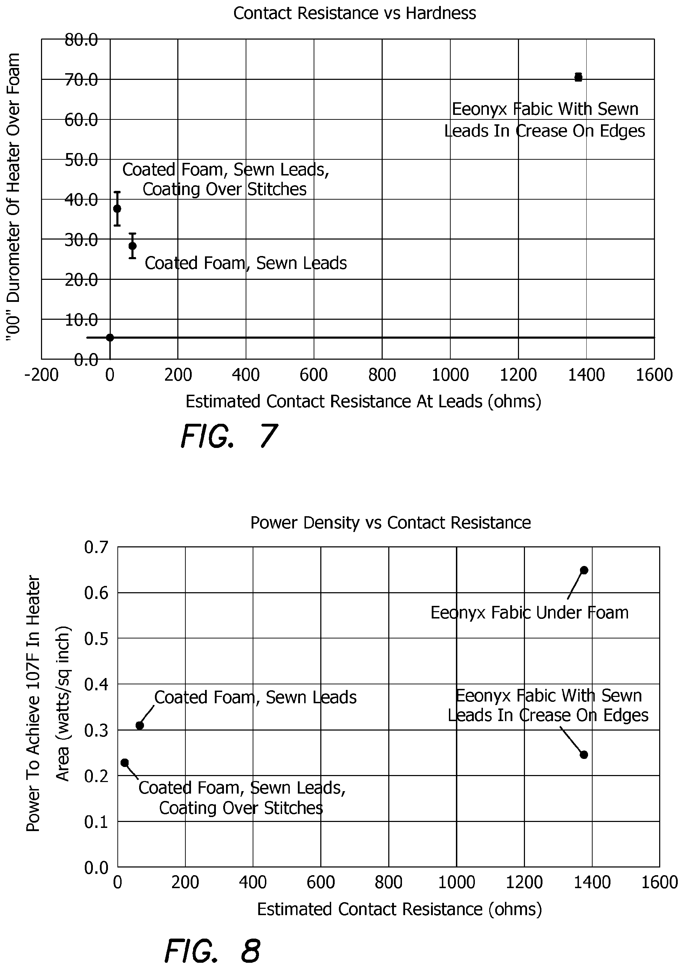

The difference in the resistance at the leads and the 4 point probe surface resistance of the Eeonyx heater is substantial and the result is a very high contact resistance for this type of heater. The fabric does not have good electrical contact with the copper leads because it is not as flexible as the foam and does not conform to the braid surface. The ideal flexible heater has very low contact resistance and very low hardness (better flexibility). The graph below shows the contact resistance vs the hardness results for the three heaters. The lowest contact resistance occurs when additional conductive coating is applied over the leads and provides additional electrical contact to the heater surface. The leads stitched on the heater foam without additional coating have slightly higher contact resistance but since the foam conforms around the copper braid, the contact resistance is still much lower than with the stiffer Eeonyx fabric heater. See Fig. 7.

The power required per unit area to heat to the desired temperature is the power density of the heater. A lower power density is more efficient and more desirable. A high contact resistance requires more voltage to reach the desired temperature of the entire heater assembly. This is not only a waste of energy, but also can heat up the leads to an unsafe temperature. The graph in Fig. 8 shows the power density vs the contact resistance of each heater system. The heating system with the lowest power density (best power utilization) is the coated foam with additional coating over the stitched leads.

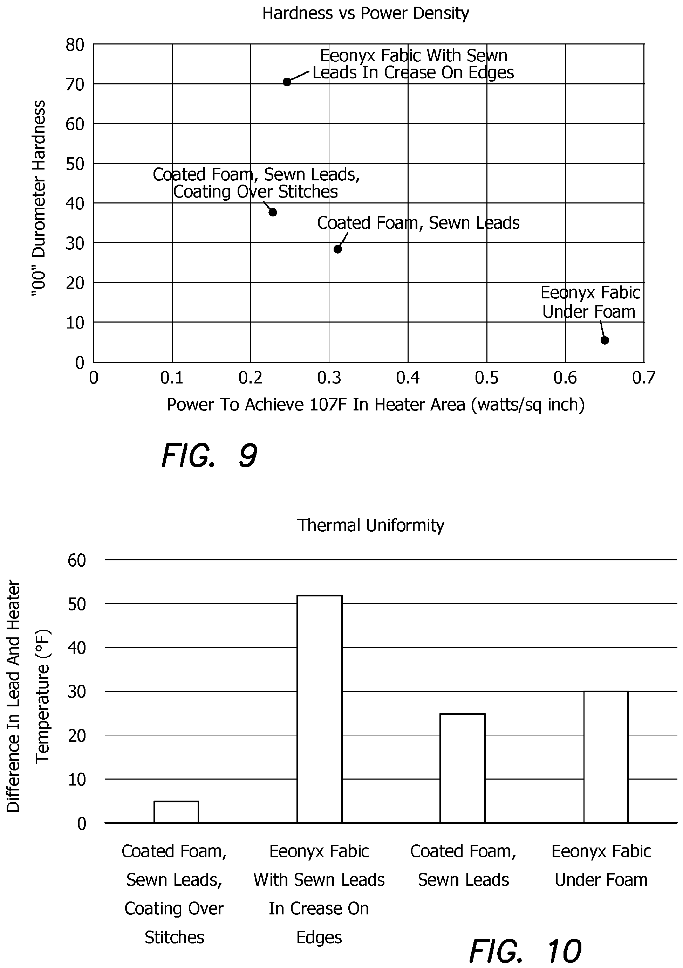

The Eeonyx fabric heater was tested under a ¼" thick piece of foam to provide more flexibility at the top surface. This strategy is described in the prior art to permit better pressure management for patient warming pads. Since the foam acts as thermal insulation, more power is required to reach the desired temperature. The graph below shows the hardness versus power density. While the Eeonyx fabric heater under the foam has the lowest hardness, it also requires the most power and is inefficient at power utilization. The coated foam samples provide both the low hardness (more flexible heater) and low power density for best heating efficiency. See Fig. 9.

Further illustrating the improvement in effective contact resistance, the temperature of the center of the heater is compared to the temperature at the copper lead using thermal imaging. Poor electric contact between the copper braid lead and the heater will increase the contact resistance and cause more current to flow into the lead increasing temperature. The graph in Fig. 10 shows the thermal uniformity of the heater examples. The best uniformity is seen with the coated foam with additional coating over the stitched leads with only 5 °F (3 °C) difference in temperature between the leads and the center of the heater area. The Eeonyx fabric heater has a difference of 52 °F (30 °C) between the leads and the center of the heater due to the poor electric connection and high contact resistance.

The graphene or graphene oxide could be used in the same technique as the carbon nanotubes if they are applied as a liquid dispersion.

Any of the resistive heaters of the invention, may, in some preferred embodiments, be further characterized as possessing one or any combination of the properties illustrated in the Examples. For example, a durometer hardness (measured according to ASTM D2240) of 10 to 50 or 20 to 40; leads attached to the foams having a contact resistance of 50 to 600 ohms, or 50 to 400 or less than 200 ohms; a 4 point probe on the surface having an average resistance of 10 to 200 ohms, or 20 to 100 ohms (or ohms per square); or a power density of 0.1 to 0.5 W/in² about 0.2 to 0.5 W/in²; or about 0.2 to 0.4 W/in² or about 0.2 to about 0.3 W/in² necessary to increase temperature from room temperature (about 75 °F (24 C)) to 107 °F (41.7 °C); and/or a temperature difference for a heater in which the electrical leads are separated by at least 7.6 cm or at least 12.7 cm (3 and 5 inches respectively) having a maximum variation of temperature between the leads and the surface of the heated area of 30 °F (16.7 °C) or less, preferably 25 °F (13.9 °C) or less, more preferably 20 °F (11.1 °C) or less, still more preferably 10 °F (5.5 °C) or less, and in some embodiments in the range of 3 to 10 °F (1.7 to 5.5 °C) or less.

## Claims

1. A method of making a resistively heatable flexible, foam, comprising:
providing a flexible, polymeric solid foam;
applying a CNT, graphene, or graphene oxide aqueous dispersion to at least one surface of the foam;
wherein the CNT, graphene, or graphene oxide aqueous dispersion penetrates into the foam;
drying the dispersion to form a CNT, graphene, or graphene oxide layer at least partly within the foam; and
applying a polymeric film over the CNT, graphene, or graphene oxide layer.

2. The method of claim 1 further comprising applying two electrical copper braid leads in electrical contact with the CNT, graphene, or graphene oxide layer; and applying additional CNT, graphene, or graphene oxide aqueous dispersion over and through the two electrical copper braid leads.

3. The method of claim 2 comprising stitching the electrical leads to the CNT, graphene, or graphene oxide layer wherein the stitches go through the CNT, graphene, or graphene oxide layer and into the foam, and wherein the stitches are nonconductive.

4. A resistively-heatable flexible, foam, comprising:
a flexible, polymeric solid foam;
a resistively-heatable CNT, graphene, or graphene oxide layer disposed on the exterior and at least partly within the foam; and
a polymeric film disposed over the CNT, graphene, or graphene oxide layer.

5. The resistively-heatable flexible, foam of claim 4, further comprising a heat releasable composition disposed within the foam and in thermal contact with the resistively-heatable CNT layer.

6. The resistively-heatable flexible, foam of claim 4, further comprising a heat releasable composition adsorbed on the polymeric film.

7. A fragrance-releasing wall plug comprising the foam of any of claims 5-6 wherein the heat releasable composition comprises a fragrance.

8. An inhaler, comprising the foam of claim 5 wherein the heat releasable composition comprises a drug or prodrug.

9. The resistively-heatable flexible, foam of claim 4, further comprising:
at least two electrical leads attached to the foam and electrically connected to the resistively-heatable layer; and further characterizable by: a surface hardness, measured according to ASTM D2240 on the surface of the heatable resistively-heatable layer, of 10 to 50 or 20 to 40; a contact resistance of 50 to 600 ohms, or 50 to 400 or less than 200 ohms when current is applied to the leads; the surface having an average surface resistance of 10 to 200 ohms, or 20 to 100 ohms; a power density of 0.016 to 0.08 W/cm²; or about 0.031 to 0.06 W/cm² or about 0.031 to 0.05 W/cm² needed to increase the average temperature of the layer from 24 °C to 41.7 °C; and/or where the electrical leads are separated by at 7.6 cm or at least 12.7 cm (3 and 5 inches respectively) and having a maximum variation of temperature between the leads and the surface of the heated area of 16.7 °C or less, preferably 13.9 °C or less, more preferably 11.1 °C or less, still more preferably 5.5 °C or less, and in some embodiments in the range of 1.7 to 5.5 °C when current is applied to the electrodes to raise the average temperature of the resistively-heatable layer to 41.7 °C.

10. The resistively-heatable flexible, foam of claim 9 where the surface of the heatable flexible foam that is nearest the heatable layer, having a surface hardness of 10 to 50.

11. The resistively-heatable flexible, foam of any of claims 9-10 wherein the leads have a contact resistance of 50 to 400 ohms when current is applied to the electrodes sufficient to raise the temperature of the layer to 41.7 °C (107 °F).

12. A method of warming a patient, comprising:
contacting the patient with the resistively-heatable foam of claim 4 or any of claims 9-11; and
applying a current through the resistively-heatable CNT, graphene, or graphene oxide layer.

13. The method of claims 1-3 wherein the aqueous dispersion comprises at least 90 mass% water, glycosaminoglycans, and carbon associated with CNTs.

14. The resistively-heatable flexible, foam of claim 9 wherein the electrical leads comprise a copper braid and wherein the stitches go through the copper braid and into the foam, wherein the stitches are nonconductive, and wherein the surface of the flexible foam conforms around the leads.

15. The resistively-heatable flexible, foam of claim 9 wherein a CNT layer penetrates in a range of 0.2 mm to 3 mm into the foam.

## Patentansprüche

1. Verfahren zum Herstellen eines widerstandsbeheizbaren flexiblen Schaums, das Folgendes umfasst:
Bereitstellen eines flexiblen Polymer-Festschaums;
Aufbringen einer wässrigen CNT-, Graphen- oder Graphenoxiddispersion auf mindestens eine Oberfläche des Schaums;
wobei die wässrige CNT-, Graphen- oder Graphenoxiddispersion in den Schaum eindringt;
Trocknen der Dispersion, um eine CNT-, Graphen- oder Graphenoxidschicht zumindest teilweise innerhalb des Schaums zu bilden; und
Aufbringen eines Polymerfilms über der CNT-, Graphen- oder Graphenoxidschicht.

2. Verfahren nach Anspruch 1, das ferner ein Aufbringen von zwei elektrischen Kupfergeflechtleitungen in elektrischem Kontakt mit der CNT-, Graphen- oder Graphenoxidschicht und ein Aufbringen von zusätzlicher wässriger CNT-, Graphen- oder Graphenoxiddispersion über den und durch die zwei elektrischen Kupfergeflechtleitungen umfasst.

3. Verfahren nach Anspruch 2, das ein Vernähen der elektrischen Leitungen mit der CNT-, Graphen- oder Graphenoxidschicht umfasst, wobei die Stiche durch die CNT-, Graphen- oder Graphenoxidschicht und in den Schaum gehen und wobei die Stiche nicht leitfähig sind.

4. Widerstandsbeheizbarer flexibler Schaum, der Folgendes umfasst:
einen flexiblen Polymer-Festschaum;
eine widerstandsbeheizbare CNT-, Graphen- oder Graphenoxidschicht, die auf der Außenseite und zumindest teilweise innerhalb des Schaums angeordnet ist; und
einen Polymerfilm, der über der CNT-, Graphen- oder Graphenoxidschicht angeordnet ist.

5. Widerstandsbeheizbarer flexibler Schaum nach Anspruch 4, der ferner eine durch Wärme freisetzbare Zusammensetzung umfasst, die innerhalb des Schaums angeordnet ist und in thermischem Kontakt mit der widerstandsbeheizbaren CNT-Schicht steht.

6. Widerstandsbeheizbarer flexibler Schaum nach Anspruch 4, der ferner eine durch Wärme freisetzbare Zusammensetzung umfasst, die an dem Polymerfilm adsorbiert ist.

7. Duftstoff freisetzender Wandstecker, der den Schaum nach einem der Ansprüche 5-6 enthält, wobei die durch Wärme freisetzbare Zusammensetzung einen Duftstoff umfasst.

8. Inhalator, der den Schaum nach Anspruch 5 enthält, wobei die durch Wärme freisetzbare Zusammensetzung ein Pharmakon oder Pro-Pharmakon umfasst.

9. Widerstandsbeheizbarer flexibler Schaum nach Anspruch 4, der ferner Folgendes umfasst:
mindestens zwei elektrische Leitungen, die an dem Schaum angebracht sind und elektrisch mit der widerstandsbeheizbaren Schicht verbunden sind; und
ferner durch Folgendes gekennzeichnet ist: eine Oberflächenhärte, gemessen nach ASTM D2240 an der Oberfläche der beheizbaren widerstandsbeheizbaren Schicht, von 10 bis 50 oder 20 bis 40; einen Kontaktwiderstand von 50 bis 600 Ohm oder 50 bis 400 oder weniger als 200 Ohm, wenn Strom an die Leitungen angelegt wird; wobei die Oberfläche einen durchschnittlichen Oberflächenwiderstand von 10 bis 200 Ohm oder 20 bis 100 Ohm aufweist; wobei eine Leistungsdichte von 0,016 bis 0,08 W/cm²; oder etwa 0,031 bis 0,06 W/cm² oder etwa 0,031 bis 0,05 W/cm² benötigt wird, um die durchschnittliche Temperatur der Schicht von 24 °C auf 41,7 °C zu erhöhen; und/oder wobei die elektrischen Leitungen durch 7,6 cm oder mindestens 12,7 cm (3 bzw. 5 Zoll) getrennt sind und eine maximale Temperaturvariation zwischen den Leitungen und der Oberfläche des beheizten Bereichs von 16,7 °C oder weniger, vorzugsweise 13,9 °C oder weniger, stärker bevorzugt 11,1 C oder weniger, noch stärker bevorzugt 5,5 C oder weniger, und bei manchen Ausführungsformen im Bereich von 1,7 bis 5,5 °C besteht, wenn Strom an die Elektroden angelegt wird, um die durchschnittliche Temperatur der widerstandsbeheizbaren Schicht auf 41,7 °C zu erhöhen.

10. Widerstandsbeheizbarer flexibler Schaum nach Anspruch 9, wobei die der beheizbaren Schicht nächstliegende Oberfläche des beheizbaren flexiblen Schaums eine Oberflächenhärte von 10 bis 50 aufweist.

11. Widerstandsbeheizbarer flexibler Schaum nach einem der Ansprüche 9-10, wobei die Leitungen einen Kontaktwiderstand von 50 bis 400 Ohm aufweisen, wenn Strom an die Elektroden angelegt wird, der ausreicht, um die Temperatur der Schicht auf 41,7 °C (107 °F) zu erhöhen.

12. Verfahren zum Erwärmen eines Patienten, das Folgendes umfasst:
Bringen des Patienten in Kontakt mit dem widerstandsbeheizbaren Schaum nach Anspruch 4 oder einem der Ansprüche 9-11; und
Anlegen eines Stroms durch die widerstandsbeheizbare CNT-, Graphen- oder Graphenoxidschicht.

13. Verfahren nach den Ansprüchen 1-3, wobei die wässrige Dispersion mindestens 90 Massen-% Wasser, Glycosaminoglykane und Kohlenstoff, der CNTs zugeordnet ist, umfasst.

14. Widerstandsbeheizbarer flexibler Schaum nach Anspruch 9, wobei die elektrischen Leitungen ein Kupfergeflecht umfassen und wobei die Stiche durch das Kupfergeflecht und in den Schaum gehen, wobei die Stiche nicht leitfähig sind und wobei die Oberfläche des flexiblen Schaums um die Leitungen herum konform ist.

15. Widerstandsbeheizbarer flexibler Schaum nach Anspruch 9, wobei eine CNT-Schicht in einem Bereich von 0,2 mm bis 3 mm in den Schaum eindringt.

## Revendications

1. Procédé de fabrication d'une mousse souple chauffable par résistance, comprenant :
la fourniture d'une mousse solide polymère souple ;
l'application d'une dispersion aqueuse de CNT, de graphène, ou d'oxyde de graphène sur au moins une surface de la mousse ;
la dispersion aqueuse de CNT, de graphène ou d'oxyde de graphène pénétrant dans la mousse ;
le séchage de la dispersion afin de former une couche de CNT, de graphène ou d'oxyde de graphène au moins en partie à l'intérieur de la mousse ; et
l'application d'un film polymère sur la couche de CNT, de graphène ou d'oxyde de graphène.

2. Procédé selon la revendication 1, comprenant en outre l'application de deux conducteurs électriques de tresse de cuivre en contact électrique avec la couche de CNT, de graphène ou d'oxyde de graphène ; et l'application d'une dispersion aqueuse supplémentaire de CNT, de graphène ou d'oxyde de graphène sur et à travers les deux conducteurs électriques de tresse de cuivre.

3. Procédé selon la revendication 2, comprenant la couture des conducteurs électriques à la couche de CNT, de graphène ou d'oxyde de graphène, les points traversant la couche de CNT, de graphène ou d'oxyde de graphène et pénétrant dans la mousse, et les points étant non conducteurs.

4. Mousse souple chauffable par résistance, comprenant :
une mousse solide polymère souple ;
une couche de CNT, de graphène ou d'oxyde de graphène chauffable par résistance disposée à l'extérieur et au moins partiellement à l'intérieur de la mousse ; et
un film polymère disposé sur la couche de CNT, de graphène ou d'oxyde de graphène.

5. Mousse souple chauffable par résistance selon la revendication 4, comprenant en outre une composition libérable par la chaleur disposée à l'intérieur de la mousse et en contact thermique avec la couche de CNT chauffable par résistance.

6. Mousse souple chauffable par résistance selon la revendication 4, comprenant en outre une composition libérable par la chaleur adsorbée sur le film polymère.

7. Prise murale libérant un parfum comprenant la mousse selon l'une quelconque des revendications 5 et 6 la composition libérable par la chaleur comprenant un parfum.

8. Inhalateur, comprenant la mousse selon la revendication 5, la composition libérable par la chaleur comprenant un médicament ou un promédicament.

9. Mousse souple chauffable par résistance selon la revendication 4, comprenant en outre :
au moins deux conducteurs électriques fixés à la mousse et reliés électriquement à la couche chauffable par résistance ; et caractérisables en outre par : une dureté de surface, mesurée selon ASTM D2240 sur la surface de la couche chauffable par résistance, de 10 à 50 ou 20 à 40 ; une résistance de contact de 50 à 600 ohms, ou de 50 à 400 ou moins de 200 ohms lorsque le courant est appliqué aux conducteurs ; la surface possédant une résistance superficielle moyenne de 10 à 200 ohms, ou de 20 à 100 ohms ; une densité de puissance de 0,016 à 0,08 W/cm² ; ou d'environ 0,031 à 0,06 W/cm² ou d'environ 0,031 à 0,05 W/cm² nécessaire pour augmenter la température moyenne de la couche de 24 °C à 41,7 °C ;
et/ou les conducteurs électriques étant séparés d'au moins 7,6 cm ou d'au moins 12,7 cm (3 et 5 pouces respectivement) et présentent une variation maximale de température entre les conducteurs et la surface de la zone chauffée de 16,7 °C ou moins, préférablement de 13,9 °C ou moins, plus préférablement de 11,1 °C ou moins, encore plus préférablement de 5,5 °C ou moins, et dans certains modes de réalisation dans la plage de 1,7 à 5,5 °C lorsqu'un courant est appliqué aux électrodes pour élever la température moyenne de la couche chauffable par résistance à 41,7 °C.

10. Mousse souple chauffable par résistance selon la revendication 9, la surface de la mousse souple chauffable qui est la plus proche de la couche chauffable, ayant une dureté de surface de 10 à 50.

11. Mousse souple chauffable par résistance selon l'une quelconque des revendications 9 à 10 les conducteurs ayant une résistance de contact de 50 à 400 ohms lorsque le courant est appliqué aux électrodes, suffisante pour élever la température de la couche à 41,7 °C (107 °F).

12. Procédé de chauffage d'un patient, comprenant :
la mise en contact du patient avec la mousse chauffable par résistance selon la revendication 4 ou l'une quelconque des revendications 9 à 11 ; et
l'application d'un courant à travers la couche de CNT, de graphène ou d'oxyde de graphène chauffable par résistance.

13. Procédé selon les revendications 1 à 3 la dispersion aqueuse comprenant au moins 90 % en masse d'eau, de glycosaminoglycanes et de carbone associés aux CNT.

14. Mousse souple chauffable par résistance selon la revendication 9, les conducteurs électriques comprenant une tresse de cuivre et les points traversant la tresse de cuivre et pénétrant dans la mousse, les points étant non conducteurs, et la surface de la mousse souple épousant les formes autour des conducteurs.

15. Mousse souple chauffable par résistance selon la revendication 9, une couche de CNT pénétrant dans une plage de 0,2 mm à 3 mm dans la mousse.
